# EUROPEAN PATENT APPLICATION

(11) **EP 1 163 928 A2**
(43) Date of publication of application: **19.12.2001**
(21) Application number: 01304408.6
(22) Date of filing: 18.05.2001
(51) Int. Cl.: A61N 1/36

(54) **Electrical clinical apparatus, electrical stimulation method and biofeedback method based on variant method**

(30) Priority: 18.05.2000 KR 2000026694
(71) Applicant: MO, Seung-Kee, Seocho-ku, Seoul (KR)
(72) Inventor: MO, Seung-Kee, Seocho-ku, Seoul (KR)
(74) Representative: Mounteney, Simon James

(57) **Abstract**

An electrical clinical apparatus and an electrical stimulation method and a biofeedback method applied thereto, which uses variants based on energy instead of a stimulation intensity, are disclosed. The electrical clinical apparatus including an electrode, which is in contact with a body part or is inserted into a body cavity, for applying electrical stimulation signals thereto, the electrical clinical apparatus comprising: a central control unit for assigning at least one of a plurality of protocols or a plurality of variants to each channel of the electrode, wherein the plurality of protocols are generated by changing electrical parameters based on an energy and the plurality of variants are generated by assembling the protocols; and an output unit for outputting the assigned protocol or variant to each channel of the electrode.

## Description

The present invention relates to an electrical stimulation method, a biofeedback method and an electrical clinical apparatus for detecting electromyogram (EMG) signals from or for applying electrical stimulation signals to said body part; and, more particularly, to an electrical stimulation method, a biofeedback method and an electrical clinical apparatus based on a variant method in embodying functions of electrical stimulation and EMG biofeedback as well as the electrical clinical apparatus including electrode to be applied in treating symptoms such as Urinary Incontinence, Sexual Dysfunction, Chronic Pelvic Pain Syndrome, Constipation and Fecal Incontinence.

An electrical clinical apparatus has been widely used in treating such dysfunction as urinary incontinence, constipation and fecal incontinence, pain relief, rehabilitation, frigidity and sexual dysfunction associated with reinnervation of damaged nerve including pelvic nerve. Such electrical clinical apparatus mostly use electromyogram (EMG) signals or current pulses because intensity of EMG is proportional to contractile force of vaginal (anal) muscle while current pulse is effective in nerve reinnervation.

Although the frequency of EMG signals mostly lie in between approx. 20 and 800 Hz and include higher frequency components than other bio signals, the electrical stimulation concept of contrary mechanism of action used only a few specific frequencies (12.5Hz and/or 50Hz) without using most of the wide range of frequency bandwidth.

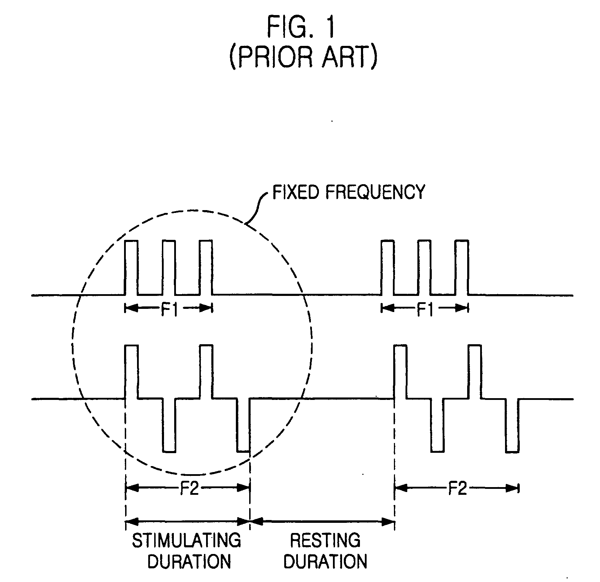

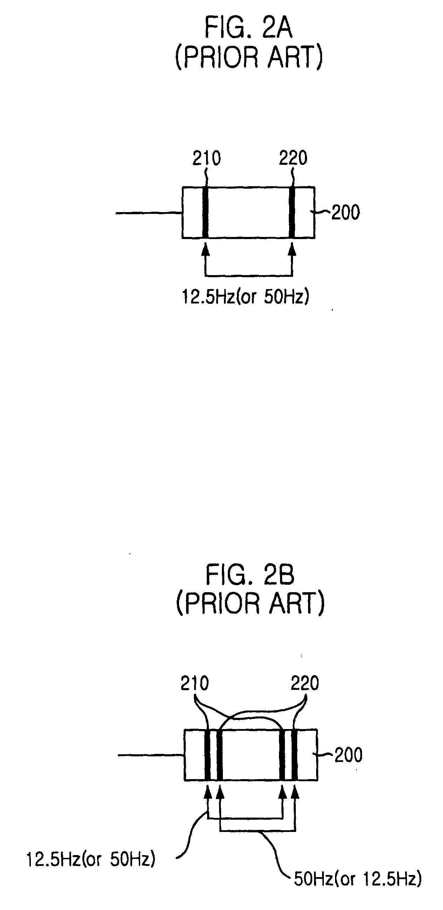

Figs. 1, 2A and 2B illustrate a conventional electrical clinical apparatus. Fig.1 illustrates frequency movements in an electrical stimulation method using a conventional frequency fixed assignment method, and Figs. 2A and 2B illustrate electrodes assigned with fixed frequency according to said electrical stimulation method.

As seen in above figures, with method of applying stimulation with a specific fixed frequency (Fl or F2) repeatedly, conventional electrical clinical apparatus fixed to a specific frequency such as 12.5 Hz or 50 Hz on a specific channel 210 or 220 of an electrode 200 used therewith. Here, the variation factor of the electrode is a regulation of electric current (voltage) based on the ratio between stimulating duration and resting duration.

However, the conventional apparatus had a problem of discomfort for the patient when the specific frequency does not fit a patient's body. Besides, even though an electrical stimulation frequency fits a patient's body, continuous stimulation onto a certain region with the same fixed frequency involves the problem of discomfort (usually one session of electrical stimulation takes 15~30 minutes). Electro-physiologically cell of the skin so easily adapt to the frequency that in the treatment thereafter the sense from electrical stimulation become dull, and thus causing discomfort to the patients. Such a problem may directly lead to discontinued exercise or discourage patient's endeavor on their treatment.

Therefore, the conventional electrical clinical apparatus had therapeutic value only in case of a patient who fits to such a frequency. It is because the patient's condition varies in terms of age, sex, race, physical constitution and so on. In short, the uniform treatment of various types of patients with only a specific fixed frequency is unreasonable in itself. The electrical stimulation has a therapeutic value of 90% or more when the stimulation fits a patient while it did not show any effects when it did not fit a patient. Accordingly, it is true that medical circles regarded electrical stimulation as a kind of inconsistent therapy

In addition, above-mentioned discomfort or inconsistent therapeutic value was pointed out as the mere side effects of the electrical stimulation in a medical publication titled "Clinical postreproductive gynecology" by Samir N. Hajj and Wendy J. Evans (Appleton & Lange, Inc., pages 308, line 25).

In addition to said problems, the EMG biofeedback of conventional electrical clinical apparatus provides only a pattern of target waveform to patients, though EMG biofeedback is used in reinforcing a specific body part with repeated exercise along with target waveform by patient. The main contributing factor in causing of tediousness is exercising with just one fixed targeted waveform, which often makes patient unable to continue pelvic muscle exercise or EMG biofeedback exercise for a sufficient time. This had been cited as a big problem. On the other hand, despite of continued exercise, exact exercise method had not been provided that exercise results were not effective and the analysis was not systematically performed.

In order to improve above-mentioned problems regarding electrical clinical apparatus with functions of electrical stimulation and EMG biofeedback to be applied in treating urinary incontinence etc., the present applicant applied for: the Korean patent application No.98-5998 filed on Feb.25, 1998, entitled "Urinary Incontinence Therapeutic Apparatus using EMG Envelope Signal"; the Korean patent application No.98-29206 filed on July 21, 1998, entitled "Electrical Apparatus for Medical Treatment using EMG Envelope Signal", claiming domestic priority based on the Korean patent application file No.98-5998; The Korean patent application No.99-4237 filed on Feb.8, 1999, entitled "Electrical Clinical Apparatus including Protective Circuit."

An object of the present invention is to provide an electrical clinical apparatus and method of embodying Variant method in electrical stimulation function for enabling consistent treatment without discomfort as well as for solving problems caused by use of only a few fixed frequencies, and EMG biofeedback function that systematically provides to the appropriate training method according to patient's unique symptom or the training needed by the patient, and the method to embody the electrical clinical apparatus that provides said functions of EMG biofeedback and electrical stimulation.

In accordance with an aspect of the present invention, there is provided an electrical clinical apparatus including an electrode, which is in contact with a body part or is inserted into a body cavity, for applying electrical stimulation signals thereto, the electrical clinical apparatus comprising: a central control unit for assigning at least one of a plurality of protocols or a plurality of Variants to each channel of the electrode, wherein the plurality of protocols are generated by changing electrical parameters based on an energy and the plurality of Variants are generated by assembling the protocols; and an output unit for outputting the assigned protocol or Variant to each channel of the electrode.

In accordance with another aspect of the present invention, there is provided an electrical stimulation method applied to an electrical clinical apparatus including a central control unit for controlling an electrode which is in contact with a body part or inserted into a body cavity for stimulating thereto by applying electrical signals, the electrical stimulation method comprising the steps of: a) generating a plurality of protocols by changing electrical parameters of the electrical signal; b) generating a plurality of variants by assembling the protocols and a plurality of reassembled variants by reassembling the variants; and c) stimulating the body part by assigning the protocols, the variants or the reassembled variants to each channel of the electrode.

In accordance with further another aspect of the present invention, there is provided an EMG biofeedback method applied to an electrical clinical apparatus including a central control unit for controlling an electrode which is in contact with a body part or inserted into a body cavity in order to stimulate thereto by applying electrical stimulation signals or to detect electromyogram (EMG) signals thereof, the EMG biofeedback method comprising the steps of: a) generating a protocol library including a plurality of protocols each having electrical parameters; b) generating a plurality of variants by assembling the protocols and a plurality of reassembled variants by reassembling the variants; and c) generating a target waveform and comparing the target waveform with an exercise waveform which results from actual exercise of a patient.
Fig. 1 shows frequency movements in an electrical stimulation method using a conventional frequency fixed assignment method;
Figs. 2A and 2B illustrate an electrode of an electrical clinical apparatus using a conventional frequency fixed assignment method;
Fig. 3 shows frequency movements in an electrical stimulation method using Variant method of the present invention;
Fig. 4 is a block diagram of the electrical clinical apparatus using Variant method according to preferred embodiment of the present invention;
Fig. 5 illustrates an example of an electrode of the electrical clinical apparatus according to preferred embodiment of the present invention;
Fig. 6 illustrates an electrode and current directions thereon in the electrical clinical apparatus of the present invention applied to neuromuscular treatments; and
Fig. 7 illustrates a target waveform to be formed by, and an exercise waveform detected by the electrical clinical apparatus of the present invention.

Hereafter, preferred embodiments of the present invention will be described in detail by referring to attached drawings.

The electrical clinical apparatus refers to the apparatus that provides functions of electrical stimulation and EMG biofeedback respectively or at the same time. Variant method of electrical stimulation is to variably assign frequency alone or together with waveform, pulse width, duty, burst time and inter-burst (rest-time). The present invention will control above parameters in the concept of energy. Variant method of EMG biofeedback is to variably assign target waveform alone or together with timing, peak, duration, endurance according to the purpose. The present patent will control the degree of symptoms or exercise levels with said 4 EMG concepts.

The electrical clinical apparatus, the object of the present invention, relates to an apparatus for medical treatment by actually detecting diversified EMG signals characteristic of each patient or on the contrary by applying patient-specific electrical stimulation signal to the treatment in reversible concept, such as apparatus for treating urinary incontinence, sexual dysfunction, chronic pelvic pain syndrome, constipation and fecal incontinence using said functions of EMG biofeedback or electrical stimulation. In addition, the present invention includes electrode adapted to contact to a patient's body part to which treatment is applied for detecting EMG signal or for applying electrical stimulation signal onto the nerve or muscle cell. Such electrode can be tubular (or rod-shape) for insertion into a body cavity to which the treatment is applied or adhesive patch type for adhering to patient's body part to which the treatment is applied. Hereafter, for the convenience of explanation, a urinary incontinence treatment apparatus will be described as an example, with focus on frequency variable assignment in electrical stimulation and with focus on target waveform in EMG biofeedback. In terms of symptoms, urinary incontinence treatment is to be explained as an example.

However, those skilled in the art will appreciate that the present invention is not limited to said frequency, target waveform and urinary incontinence treatment, but applied to electrical stimulation parameters as stimulation waveform, pulse width, duty, burst time, inter-burst time (rest time) etc, or to EMG biofeedback parameters as timing, peak, duration, endurance etc, or to stand-alone or assembled clinical apparatus using electrical/magnetic conversion and so on.

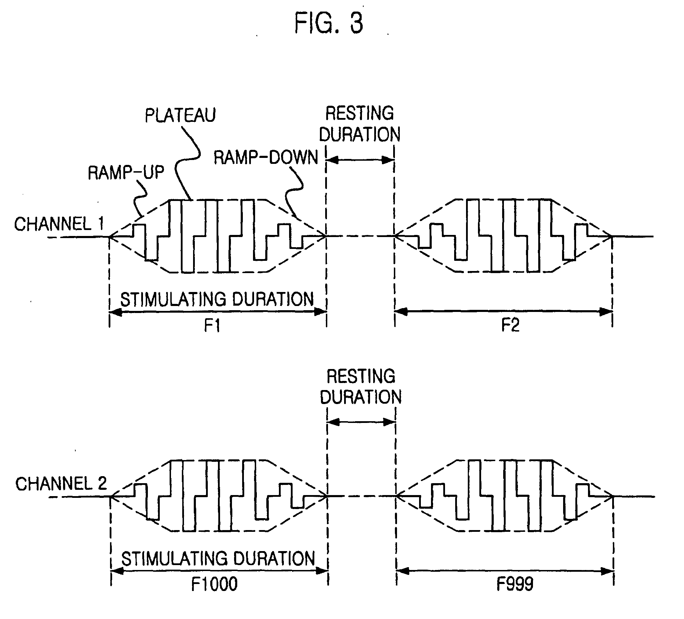

Fig. 3 shows a frequency movement of electrical clinical apparatus through frequency Variant assignment when using a Variant method of the present invention. As illustrated in above drawing, the frequency bandwidth to be assigned on each channel may vary within the range of F1- F1000(1-1000Hz)

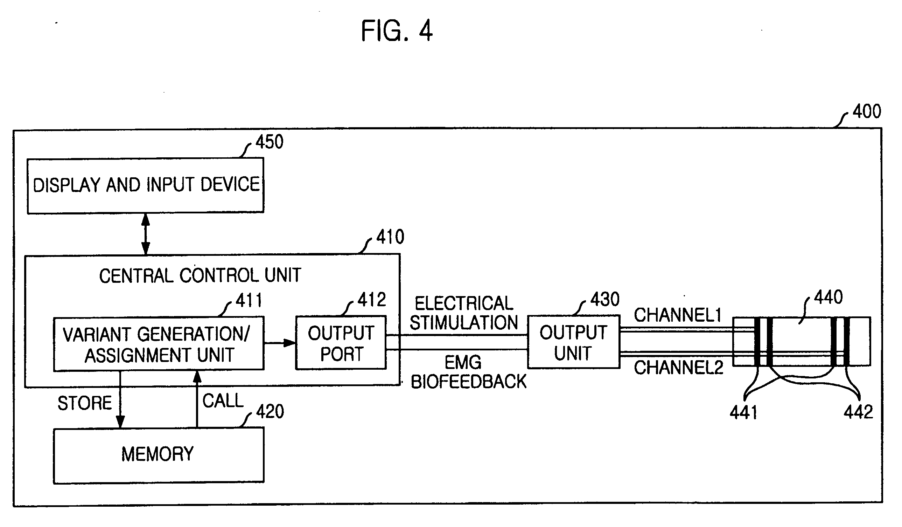

Fig. 4 is a block diagram of the electrical clinical apparatus using Variant method using the frequency Variant assignment method according to preferred embodiment of the present invention.

As illustrated in Fig. 4, the electrical clinical apparatus 400 according to the present invention can include a central control device 410, a memory 420, an output circuit 430, an electrode 440 and a display and input unit 450.

The central control device 410 generates a protocol library of which the protocols electric signals are different from each other in terms of frequency, pulse width, duty ratio, intensity/voltage of supplied electric current, burst duration and inter-burst duration. The variable frequency, pulse width, duty ratio, intensity/voltage of the supplied electric current of electric signal may be pertinently settable according to the treatment purposes.

A user selects stimulation intensities of a first and a second channel of the electrode through a variable output device of an output port 412 so as to be appropriate to himself/herself. In other words, values of the frequency, the pulse width, the duty, the voltage/current intensity, the burst duration and the inter-burst duration which the user selects are received through an input device of a display and input unit 450, confirmed by the user and transmitted to the variant generation/assignment unit 411.

The frequency, the pulse width, the duty, the voltage/current intensity, the burst duration and the inter-burst duration are used as variable numbers of parameters deciding the energy value. Maximum output energy per pulse can be expressed by equation (1) as:$\text{P} \text{×} {\text{t}}_{\text{PW}} \text{=} {\text{V}}_{\text{m}} \text{×} {\text{I}}_{\text{m}} \text{×} {\text{t}}_{\text{PW}} \text{=} {\text{I}}_{\text{m}} {\text{}}^{\text{2}} \text{×} {\text{R}}_{\text{s}} \text{×} {\text{t}}_{\text{PW}} \text{=} {\text{V}}_{\text{m}} \text{×} \text{Q} \text{[} \text{mJ/pulse} \text{]}$ where,$\text{Q} \text{= 2 ×} \frac{{\text{V}}_{\text{m}}}{{\text{R}}_{\text{s}}} \text{×} {\text{t}}_{\text{PW}} \text{[} \text{µC/pulse} \text{]}$ (in case of bi-phasic wave),$\text{Q} \text{=} \frac{{\text{V}}_{\text{m}}}{{\text{R}}_{\text{s}}} \text{×} {\text{t}}_{\text{PW}} \text{[} \text{µC/pulse} \text{]}$ (in case of mono-phasic wave),${\text{V}}_{\text{m}} \text{=} \frac{{\text{V}}_{\text{rms}}}{\text{Duty}} \text{=} \frac{{\text{I}}_{\text{rms}} \text{×} {\text{R}}_{\text{s}}}{\text{Duty}} \text{=} \frac{{\text{V}}_{\text{p-p}}}{\text{2}} \text{[} \text{V} \text{],}$$\text{Duty=2×f×} {\text{t}}_{\text{PW}} \text{,}$${\text{Energy per channel = V}}_{\text{m}} \text{×} {\text{Q × t}}_{\text{B}} \text{,}$

*t*_{*PW*} denotes a pulse width, *V*_{*m*} a maximum voltage intensity, *I*_{*m*} a maximum current intensity, *R*_{*s*} a skin resistance, Q a charge and f frequency.

Even if the energy of the output signal has the same value, parameters included in the energy can be different from each other. In other words, increase/decrease in the value of each parameter results in keeping a constant value. Therefore, the electrical stimulation having the same amount energy but different parameters (which is referred to as a 'protocol') provides different feelings and stimulation characteristics to the user. An example of protocols is described in table 1.

Referring to the table 1, the protocols 1 to n have the same values in the energy, i.e., 2.0[mJ]. However, each of the protocols includes different values in the intensity, the frequency, the duty, the pulse width and the burst duration from each other.

When the user wants to re-adjust the intensities of the first and the second channels of the electrode, the processes are performed as the same method.

For example, it may be settable at an energy of 1 to 20 mJ, a maximum voltage of 1 to 30 Vm, a frequency bandwidth of 1 to 1000Hz, a duty of 0 to 40%, a pulse width of 10 to 1000µ sec, a burst duration of 0.1 to 30 sec, and a wave form of a biphasic wave, a mono-phasic wave, a rectangular wave or a triangular wave. The inter-burst time ranges between 0 and 30 min as including the burst duration. The burst duration includes Ramp-Up time, Plateau time and Ramp-Down time. Table 1 shows an example of the protocol library.

**[Table 1]**

| LIBRARY | ENERGY [mJ] | INTENSITY **[Vm]** | FREQUENCY [Hz] | DUTY [%] | PULSE WIDTH [µsec] | BURST TIME [sec] | WAVEFORM |
|---|---|---|---|---|---|---|---|
| PROTOCOL1 | 2.0 | 10 | 10 | 1.0 | 1000 | 5 | BIPHASIC WAVE |
| PROTOCOL2 | 2.0 | 15 | 20 | 1.1 | 556 | 4 | BIPHASIC WAVE |
| PROTOCOL3 | 2.0 | 20 | 30 | 1.2 | 417 | 3 | BIPHASIC WAVE |
| PROTOCOL4 | 2.0 | 25 | 40 | 1.6 | 400 | 2 | BIPHASIC WAVE |
| PROTOCOL5 | 2.0 | 30 | 50 | 2.7 | 556 | 1 | BIPHASIC WAVE |
| PROTOCOL6 | 2.0 | 30 | 60 | 0.5 | 93 | 6 | BIPHASIC WAVE |
| PROTOCOL7 | 2.0 | 28 | 70 | 0.6. | 92 | 7 | BIPHASIC WAVE |
| PROTOCOL8 | 2.0 | 40 | 80 | 0.3 | 40 | 8 | BIPHASIC WAVE |
| PROTOCOL9 | 2.0 | 45 | 90 | 0.2 | 28 | 9 | BIPHASIC WAVE |
| PROTOCOL10 | 2.0 | 12 | 100 | 3.4 | 348 | 10 | BIPHASIC WAVE |
| PROTOCOL11 | 2.0 | 23 | 200 | 19.0 | 950 | 1 | BIPHASIC WAVE |
| PROTOCOL12 | 2.0 | 27 | 300 | 10.3 | 345 | 2 | BIPHASIC WAVE |
| PROTOCOL13 | 2.0 | 35 | 400 | 5.4 | 137 | 3 | BIPHASIC WAVE |
| PROTOCOL14 | 2.0 | 37 | 500 | 4.6 | 92 | 4 | BIPHASIC WAVE |
| PROTOCOL15 | 2.0 | 11 | 600 | 49.8 | 830 | 5 | BIPHASIC WAVE |
| ······ | ······ | ······ | ······ | ······ | ······ | ······ | ······ |
| PROTOCOL n | 1~20 | 1~50 | 1~1000 | 0~40 | 10~1000 | 0.1~30 | BIPHASIC WAVE, MONOPHASIC WAVE, SQUARE WAVE, TRIANGULAR WAVE |

Variant electrical stimulation refers to protocol components, i.e., the frequency, the duty, the pulse width, the burst time and the wave form to be repeated at least more than one time or assembly of more than two protocols.

A lot of protocols can be generated besides the protocols in table 1 in accordance with variations of the frequency, the duty, the pulse width, the burst time and the waveform. As described above, the protocols are generated by selecting various values of the frequency, the duty, the pulse width, the burst time and the wave form within a range of energy 1~20 based on equation 1. Even though it has the same energy value, each protocol or Variant having different parameters differently affects on humans' body. The Variants of electrical stimulation also may be assembled each other into reassembled variants. The protocol, the Variant resulted from an assembly of the protocols and reassembled variants resulted from the assembly of the Variants can be generated and assigned without any limitation.

As describing generation of protocols, a protocol generation program is fetched from a storage device and installed on a read only memory (RAM), protocols having a certain energy are generated by using electrical parameters of the electrical signal.

The protocols are assembled in the variant generation/assignment unit, thereby generating a plurality of variants. The variants may be reassembled, thereby generating another plurality of assembled variants. The protocols, the variants and the reassembled variants are serially assigned to each channel of the electrode 440. The electrical parameters of the protocol, which are used in the generation of the protocol, are updated on the RAM and used as data of the electrical stimulation signal.

Such various protocols thus formed are to be assembled into Variants in the Variant generation/assignment section 411 of the central control device 410, or the Variants are to be assembled into combinations of the Variants. Each channel of the electrode 440 is to be assigned with the generated protocols, Variants and combinations of Variants.

The data of the electrical stimulation signal are stored a memory device 420. The memory device may be a RAM or a hard disk driver (HDD).

A display and input unit 450 includes a CRT monitor and a keyboard in case of a personal computer, a LCD/LED monitor and a keyboard in case of a micro-computer (MICOM). The variant generation/assignment unit 411 includes a central processing unit (CPU) of the personal computer or a micro-controller of the MICOM. The memory device includes a HDD, a floppy disk driver (FDD) or a RAM in case of the personal computer, or a ROM, a RAM or a flash memory in case of the MICOM.

After the generation and assignment of the protocols and the variants are completed, a clinical process is selected. In this embodiment, the protocols and the variants are generated in the electrical clinical apparatus. However, the electrical clinical apparatus may only selects and uses the protocols and the variants which are generated, assigned and stored on the memory device when being manufactured, or receives the protocols and the variants from an external memory device through a serial port, a parallel port or a wireless port.

When the clinical program is selected, the protocols and the variants corresponding to a predetermined energy are transmitted to the output port 412. The output port provides ports for signal processing of a first and a second channel, and outputs TTL level value in hardware.

The output unit 430 includes a digital/analog (D/A) converter, an amplifier for amplifying the TTL level value to a predetermined intensity (1~30[Vm]) and a variable output device for controlling an intensity of the output appropriate to the user.

The stimulation signal outputted from the output unit 430 is applied to patient's body through the electrode 440.

For convenience of explanation, the preferred embodiment according to the present invention supposes that the electrode 440 is insertion type with two channels thereon. However, those skilled in the art will appreciate that channels of electrode can be pertinently configured.

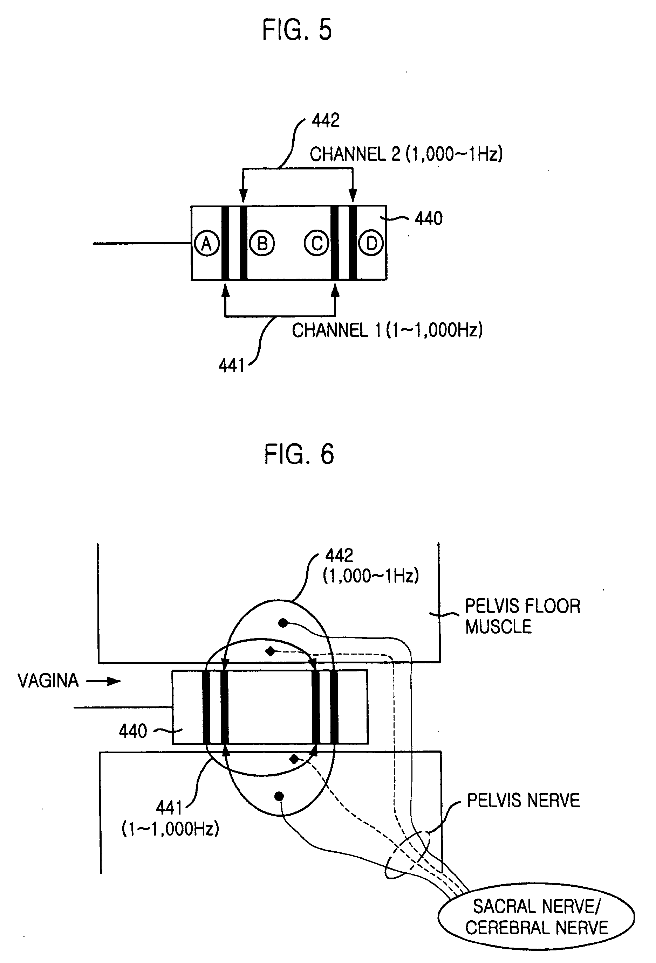

As illustrated in Fig.5, the electrode 440 includes a first channel 441 and a second channel 442, and such channels can be configured with one or combined electric currents in the same direction or opposite direction. For example, the first channel 441 may be configured with A and D, A and C, or A and B while the second channel 442 can be configured with D and B, D and A, or D and C.

Hereafter, a method of assigning the first channel 441 and the second channel 442 of the electrode 440 with the protocols and Variants will be described below.

If 120 or more protocols are to be generated, they will be serially applied during typically 30 minutes of an electrical stimulation session. For example, the first channel 441 is to be assigned with protocol 1, the second channel 442 is to be assigned with protocol 15 to protocol 2, and thus the first and second channels 441 and 442 are serially applied with the protocols different from each other. In addition to the method of assigning the two channels with the protocols different from each other, the same protocols may be assigned on both channels simultaneously. Table 2 shows an example of the assignment method of the protocols.

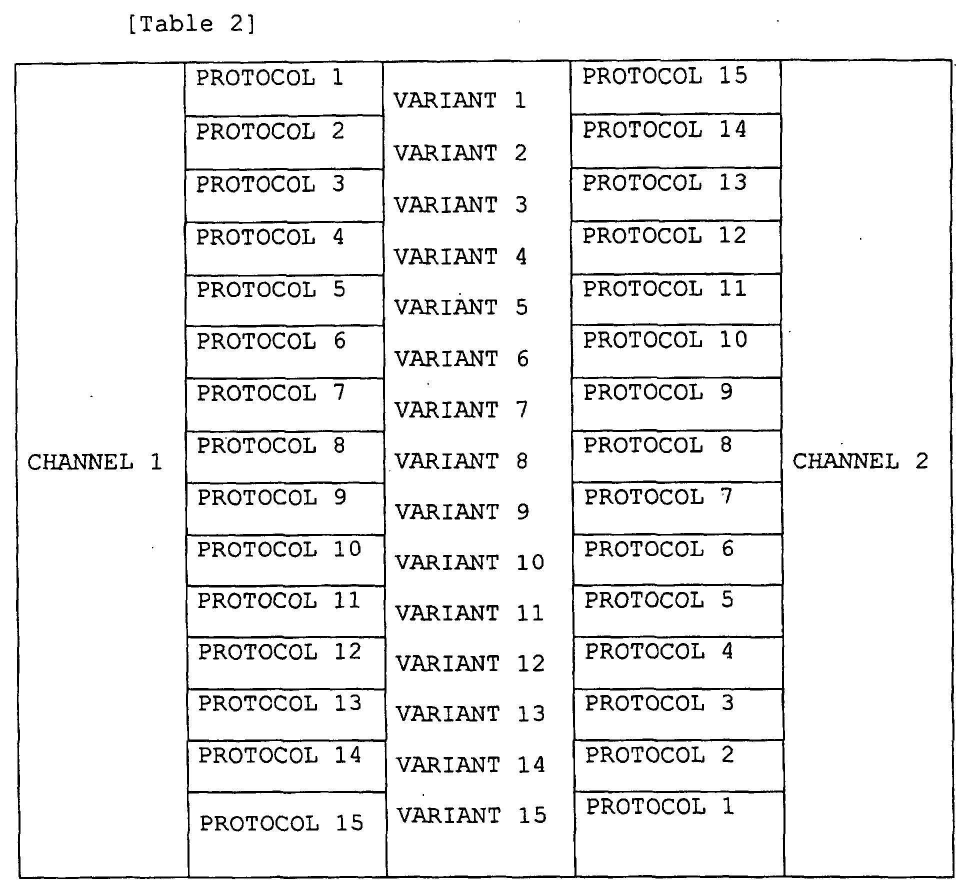

Here, the Variant of electrical stimulation is to be formed by assembling the protocols which are different from each other.

The energy of Variant of electrical stimulation can be calculated with electrical parameters such as waveform, frequency, pulse width, duty, burst time and inter-burst time (rest time). Various protocols can be generated by summation or difference of energy to be assigned on each channel. The Variants of electrical stimulation also may be assembled each other into reassembled variants. Variant electrical stimulation method refers to the method of finding the most appropriate electrical parameters to a patient for one session (one treatment cycle), wherein the minimum unit of applied Variant electrical stimulation is to be referred as Variant protocol of electrical stimulation.

Those Variants may be alternately assigned to each channel. The method of assigning channels with Variants will be described as follows.

For example, the first channel is assigned with the Variants of high frequency while the second channel is assigned with the Variants of low frequency. Otherwise, the first channel is assigned with the Variants of wide pulse width, while the second channel is assigned with the Variants of narrow pulse width. That is, by assigning each channel of the electrode with signals of such opposite frequency elements, energy applied to each channel of the electrode may be made different. In this case, the same results may be obtained by applying electrical stimulation onto the first and second channels 441 and 442 after changing waveform, duty ratio, intensity, burst time, inter-burst time (rest time) etc. The electrical stimulation is applied after serially assigning the first and second channels 441 and 442 with the Variants different from each other until one session of treatment has been finished. Each channel may be assigned with one of the Variants or combination of Variants. Accordingly, all the Variants to be applied may be configured in a form of Variant 1 + Variant 2 +...+ Variant n, and table 3 shows such an example.

**[Table 3]**

| CONFIGURATION | CHANNEL 1 | CHANNEL 2 |
|---|---|---|
| STIMULATION NO. 1 | VARIANT | - |
| STIMULATION NO. 2 | - | VARIANT |
| STIMULATION NO. 3. | VARIANT | VARIANT |

Functions of the Variants may be applied to each channel of the electrode alternately or simultaneously. In case where stimulation serially applied to the body during one treatment session of about 15~30 minutes in total, such characteristics of Variants internally carry out electrophysiological functions of reinnervating pelvic floor muscle nerve damaged due to childbirth and so on without causing any feeling of discomfort to patients. Such a phenomenon may provide unique feeling of the rhythmical effects of massage by the extremely deft and swift hands combined with electrophysiological effects peculiar to such electrical stimulation.

Here, the protocols having the Variants are assigned not with a specific frequency but with wide range of frequency bandwidth of 1 to 1000Hz impartially distributed, especially with emphasis on low frequency bandwidth of 1 to 200 Hz fit for human body. The reason is that effective frequencies are not fixed in a specific frequency even though most of them are distributed in relatively low bandwidth. That is, within 1~1000Hz bandwidth, especially within low frequency bandwidth of 1~200Hz, the frequencies induce contraction and relaxation of pelvic floor muscle as well as reinnervation of the nerve thereof, not being limited to a specific frequency. Accordingly, the stimulation of pelvic floor muscle with such frequencies may cover almost all the frequencies of patients so that consistent and stable treatment results may be obtained.

Once protocols or Variants to be assigned on each channel of the electrode are determined according to above-mentioned method, they are stored in the memory device 420 linked to the central control device 410. According to a fixed program, by a calling of the central control device 410, protocols and Variants are transmitted again to the Variant generation/assignment section 411 to be assigned on an output port 412 linked therewith, and then are to be transmitted to be assigned on each channel of electrode 440 through the output circuit 430 linked to the central control device 410. Thus, the first and second channels 441 and 442 of the electrode 440 finally receive the amplified signals by hardware in the output circuit 430. As illustrated in Fig. 6, the electrode, Also, Fig.6 illustrates how the electrode applies the stimulation onto the nerve and muscle of vagina by contacting thereto as an embodiment of the electrical stimulation method of the present invention.

Those skilled in the art will know that the present invention may be modified or amended in the context of a technical concept of the present invention. For example, the protocol library may be downloaded into the electrical clinical apparatus of the present invention, not in the method controlled by the incorporated central control device, but downloaded or used in hook-up with externally connected communication equipment or computer. Besides, the electrical clinical apparatus of the present invention may additionally includes a display device which displays a progress of electrical stimulation using graphic, character or small lamp and so on.

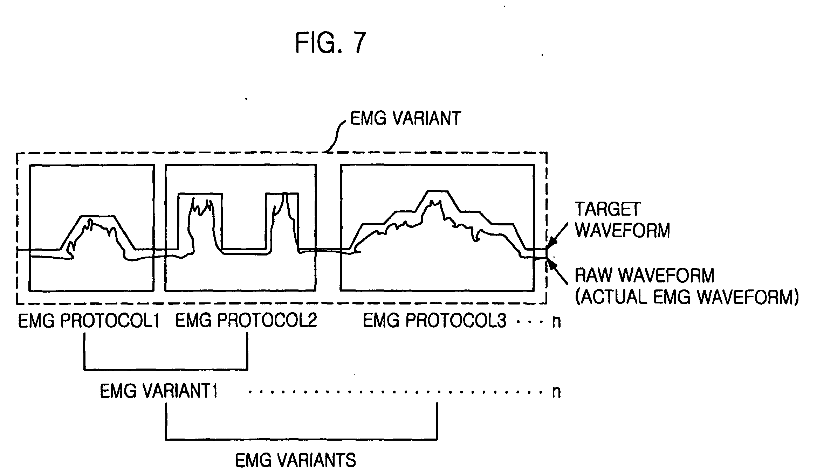

As illustrated in Fig. 7, the EMG biofeedback of electrical clinical apparatus of the present invention may raise the treatment effects of EMG biofeedback function by inducing patients to match exercise waveforms to the target waveforms by concurrently displaying the two waveforms on the display device comprised in the electrical clinical apparatus, wherein the exercise waveform (or raw waveform) is to be the EMG signals detected as a result of the patient's actual exercise while the target waveform is to be selected from the protocol library including the EMG protocols (EMG protocol 1, EMG protocol 2, ... , EMG protocol n) formed by the variation of target waveforms for patients to watch, follow and exercise in order to set the EMG signals detected from a body part of patient by said electrode as a guidance for patient to exercise. It is advisable to compose the target waveform with only signals of desired characteristics out of the EMG protocols or Variants formed by assembling the EMG protocols, or out of reassembled variants. The signal characteristics of EMG Variants include timing, peak, duration and endurance. Hence, EMG Variants can be arbitrarily formed by assembling protocols depending on exercise target. In more detail, timing is the method to contract desired muscle prior to undesired muscle. Peak is the method to train the periodic contractile force to the maximum intensity. Duration is the method to maintain the contractile duration of desired muscle to the maximum length. Endurance is the method to maintain maximum possible length of duration at the maximum strength.

The EMG Variant is to be reassembled to contain opposite or different types of 4 EMG elements each other. The specific EMG Variant can be assembled with Timing, Peak, Duration and Endurance according to symptoms and exercise purposes. The EMG Variants also may be reassembled each other into reassembled variants. Variant EMG biofeedback method refers to the method of help using the most appropriate EMG exercise pattern to a patient for one session (one exercise cycle), wherein the minimum unit of applied Variant EMG biofeedback is to be referred as Variant protocol of EMG biofeedback.

In the treatment of pelvic floor muscle damaged from childbirth and so on, the electrical clinical apparatus embodied by Variant technology of the present invention can reinnervate the pelvic floor muscle nerve without causing any pain or discomforts. The electrical stimulation applied in the present invention, adjusting electrical stimulation parameters in terms of energy concept, can provide the effects of consistent and stable treatment results to most people. Besides, in the EMG biofeedback, the present invention may get the effects of scientifically improving the exercise results by providing target waveforms desired or needed in a systematic manner.

Although the preferred embodiments of the invention have been disclosed for illustrative purposes, those skilled in the art will appreciate that various modifications, additions and substitutions are possible, without departing from the scope and spirit of the invention as disclosed in the accompanying claims.

## Claims

1. An electrical clinical apparatus including an electrode, which is in contact with a body part or is inserted into a body cavity, for applying electrical stimulation signals thereto, the electrical clinical apparatus comprising:
a central control means for assigning at least one of a plurality of protocols or a plurality of Variants to each channel of the electrode, wherein the plurality of protocols are generated by changing electrical parameters based on an energy and the plurality of Variants are generated by assembling the protocols; and
an output means for outputting the assigned protocol or Variant to each channel of the electrode.

2. The electrical clinical apparatus recited in claim 1, further comprising:
a storage means for storing the plurality of protocols or Variants.

3. The electrical clinical apparatus recited in claim 2, further comprising:
a user input device for receiving a selection signal selecting at least one of the plurality of protocols or Variants from a user.

4. The electrical clinical apparatus recited in claim 3, wherein the electrical parameter includes a waveform, a pulse width, a duty, a voltage/current intensity, a burst duration and an inter-burst time of an electrical signal.

5. The electrical clinical apparatus recited in claim 4, wherein a maximum output energy per a pulse can be obtained by an equation as:$\text{P} \text{×} {\text{t}}_{\text{PW}} \text{=} {\text{V}}_{\text{m}} \text{×} {\text{I}}_{\text{m}} \text{×} {\text{t}}_{\text{PW}} \text{=} {\text{I}}_{\text{m}} {\text{}}^{\text{2}} \text{×} {\text{R}}_{\text{s}} \text{×} {\text{t}}_{\text{PW}} \text{=} {\text{V}}_{\text{m}} \text{× Q} \text{[} \text{mJ/pulse} \text{]}$ where,$\text{Q} \text{= 2 ×} \frac{{\text{V}}_{\text{m}}}{{\text{R}}_{\text{s}}} \text{×} {\text{t}}_{\text{PW}} \text{[} \text{µC/pulse} \text{]}$ (in case of bi-phasic wave),$\text{Q} \text{=} \frac{{\text{V}}_{\text{m}}}{{\text{R}}_{\text{s}}} \text{×} {\text{t}}_{\text{PW}} \text{[} \text{µC/pulse} \text{]}$ (in case of mono-phasic wave),${\text{V}}_{\text{m}} \text{=} \frac{{\text{V}}_{\text{rms}}}{\text{Duty}} \text{=} \frac{{\text{I}}_{\text{rms}} \text{×} {\text{R}}_{\text{s}}}{\text{Duty}} \text{=} \frac{{\text{V}}_{\text{p-p}}}{\text{2}} \text{[} \text{V} \text{].}$$\text{Duty=2×f×} {\text{t}}_{\text{PW}} \text{,}$${\text{Energy per channel = V}}_{\text{m}} \text{×} \text{Q} \text{×} {\text{t}}_{\text{B}} \text{,}$ *t*_{*PW*} denotes a pulse width, *V*_{*m*} a maximum voltage intensity, *I*_{*m*} a maximum current intensity, *R*_{*s*} a skin resistance,Q a charge and f frequency.

6. The electrical clinical apparatus recited in claim 5, wherein the central control means includes:
a Variant generation means for generating the plurality of protocols by changing the electrical parameters based on the energy; and
a Variant assignment means for assigning the protocol or the Variant to each channel in accordance with a control signal.

7. The electrical clinical apparatus recited in claim 6, wherein the protocol or the Variant can includes magnetic parameters having the same energy as the electrical parameter.

8. The electrical clinical apparatus recited in claim 7, wherein the electrode includes at least one channel, and a current on each channel has the same or an opposite direction from each other.

9. The electrical clinical apparatus recited in claim 8, wherein the protocol or the Variant assigned to each of the channels is opposite or different from each other.

10. The electrical clinical apparatus recited in claim 9, further including:
a display unit for displaying a progress of an electrical stimulation.

11. The electrical clinical apparatus recited in claim 10, wherein the central control unit further includes:
an output port for outputting the protocol or the variant to the output means.

12. The electrical clinical apparatus recited in claim 10, wherein the electrode includes an insertion type electrode, a patch type electrode or a spatial type electrode.

13. The electrical clinical apparatus recited in claim 12, wherein the central control unit serially assigns each channel with a different protocol, variant or reassembled variant formed by reassembling the variants or alternately assigns the same protocols or the Variants or the reassembled variants to each channel.

14. The electrical clinical apparatus recited in claim 13, wherein a frequency bandwidth of the electrical signal is at a range of 1 to 1000Hz.

15. The electrical clinical apparatus recited in claim 14, wherein the storage means is located in the electrical clinical apparatus.

16. The electrical clinical apparatus recited in claim 14, wherein the storage means is located in an outside of the electrical clinical apparatus.

17. The electrical clinical apparatus recited in claim 16, wherein the protocols or the Variants stored in the storage means are received through a serial port, a parallel port or a wireless port.

18. An electrical stimulation method applied to an electrical clinical apparatus including a central control unit for controlling an electrode which is in contact with a body part or inserted into a body cavity for stimulating thereto by applying electrical signals, the electrical stimulation method comprising the steps of:
a) generating a plurality of protocols by changing electrical parameters of the electrical signal;
b) generating a plurality of variants by assembling the protocols and a plurality of reassembled variants by reassembling the variants; and
c) stimulating the body part by assigning the protocols, the variants or the reassembled variants to each channel of the electrode.

19. The electrical stimulation method as recited in claim 18, further comprising the step of:
d) storing the protocols, the variants or the reassembled variants onto a storage means.

20. The electrical stimulation method as recited in claim 18, further comprising the step of:
e) receiving a selection signal of selecting at least one of the protocols, the variants or the reassembled variants.

21. The electrical stimulation method as recited in claim 20, further comprising the step of:
f) displaying a progress of the electrical stimulation on a display unit.

22. The electrical stimulation method as recited in claim 21, wherein the electrical parameter includes a waveform, a pulse width, a duty, a voltage/current intensity, a burst duration and an inter-burst time of the electrical signal.

23. The electrical stimulation method as recited in claim 22, wherein the electrode includes at least one channel.

24. The electrical stimulation method as recited in claim 23, wherein a current on each channel has the same or opposite direction from each other.

25. The electrical stimulation method as recited in claim 23, wherein the display unit is incorporated in or linked externally with the electrical clinical apparatus.

26. The electrical stimulation method as recited in claim 24, wherein the step c) includes the step of:
serially assigning the protocols, the variants or the reassembled variant to each channel of the electrode.

27. The electrical stimulation method as recited in claim 24, wherein the step c) includes the step of:
alternately assigning the same protocols, the variants or the reassembled variant to different channels of the electrode.

28. The electrical stimulation method as recited in claim 24, wherein the electrode includes an insertion type electrode, a patch type electrode or a spatial type electrode.

29. The electrical stimulation method as recited in claim 28, wherein a frequency bandwidth of the electrical signal is at a range of 1 to 1000Hz.

30. The electrical stimulation method as recited in claim 29, wherein the storage means is located in the electrical clinical apparatus.

31. The electrical stimulation method as recited in claim 29, wherein the storage means is located in an outside of the electrical clinical apparatus.

32. The electrical stimulation method as recited in claim 31, wherein the protocol or the Variant stored in the memory is received through a serial port, a parallel port or a wireless port.

33. An EMG biofeedback method applied to an electrical clinical apparatus including a central control unit for controlling an electrode which is in contact with a body part or inserted into a body cavity in order to stimulate thereto by applying electrical stimulation signals or to detect electromyogram (EMG) signals thereof, the EMG biofeedback method comprising the steps of:
a) generating a protocol library including a plurality of protocols each having electrical parameters;
b) generating a plurality of variants by assembling the protocols and a plurality of reassembled variants by reassembling the variants; and
c) generating a target waveform and comparing the target waveform with an exercise waveform which results from actual exercise of a patient.

34. The EMG biofeedback method as recited in claim 33, wherein the target waveform includes a timing, a peak, a duration and an endurance of the electrical signal.

35. The EMG biofeedback method as recited in claim 34, wherein the electrode includes at least one channel.

36. The EMG biofeedback method as recited in claim 35, wherein the electrode includes an insertion type electrode, a patch type electrode or a spatial type electrode.

37. The EMG biofeedback method as recited in claim 36, further including the step of:
d) storing the plurality of protocols, the variants or the reassembled variants onto a storage means.

38. The EMG biofeedback method as recited in claim 37, further including the step of:
e) displaying a progress of an EMG biofeedback on a display unit.

39. The EMG biofeedback method as recited in claim 38, wherein the step e) includes the step of:
simultaneously displaying the target waveform and the exercise waveform.

40. The EMG biofeedback method as recited in claim 39, wherein the electrical stimulation signal is an EMG triggered electrical stimulation signal in which an electrical stimulation is applied when,patent's muscle contracted above a certain intensity level in actual EMG biofeedback exercise.

41. The EMG biofeedback method as recited in claim 40, wherein the storage means is located in the electrical clinical apparatus.

42. The EMG biofeedback method as recited in claim 41, wherein the storage means is located in an outside of the electrical clinical apparatus.

43. The EMG biofeedback method as recited in claim 42, wherein the protocol or the variant stored in the storage means is received through a serial port, a parallel port or a wireless port.

44. The EMG biofeedback method as recited in claim 43, wherein the timing, the peak, the duration and the endurance of the electrical signal are detected based on superconductivity instead of the electromyography (EMG).

45. An electrical stimulation method, substantially as hereinbefore described with reference to Figure 3 of the accompanying drawings.

46. An electrical stimulation apparatus, substantially as hereinbefore described with reference to Figures 4, 5 and 6 of the accompanying drawings.
